(19) Europäisches Patentamt
European Patent Office
Office européen des brevets

(11) **EP 3 492 089 B1**

(12) **EUROPÄISCHE PATENTSCHRIFT**

(45) Veröffentlichungstag und Bekanntmachung des Hinweises auf die Patenterteilung:
**17.02.2021  Patentblatt 2021/07**

(51) Int Cl.:
*A61K 35/00* (2006.01)   *A61K 35/74* (2015.01)
*A61P 17/00* (2006.01)

(21) Anmeldenummer: **17204458.8**

(22) Anmeldetag: **29.11.2017**

(54) **DERMATOLOGISCHE ZUSAMMENSETZUNG ENTHALTEND ESCHERICHIA COLI UND ENTEROCOCCUS FAECALIS**

DERMATOLOGICAL COMPOSITION CONTAINING ESCHERICHIA COLI AND ENTEROCOCCUS FAECALIS

COMPOSITION DERMATOLOGIQUE CONTENANT ESCHERICHIA COLI ET ENTEROCOCCUS FAECALIS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.06.2019  Patentblatt 2019/23**

(73) Patentinhaber: **SymbioGruppe GmbH & Co. KG
35745 Herborn (DE)**

(72) Erfinder:
• **Müller, Hans-Jörg
  35619 Braunfels (DE)**
• **Schmidts, Thomas Michael
  35396 Gießen (DE)**
• **Zimmermann, Kurt
  35745 Herborn (DE)**
• **Rusch, Volker
  35745 Herborn (DE)**

(74) Vertreter: **Isarpatent
Patent- und Rechtsanwälte Behnisch Barth Charles
Hassa Peckmann & Partner mbB
Friedrichstrasse 31
80801 München (DE)**

(56) Entgegenhaltungen:
**DE-T2- 69 019 956**

• SUSANNE LAU ET AL: "Oral application of bacterial lysate in infancy decreases the risk of atopic dermatitis in children with 1 atopic parent in a randomized, placebo-controlled trial", JOURNAL OF ALLERGY AND CLINICAL IMMUNOLOGY, ELSEVIER, AMSTERDAM, NL, Bd. 129, Nr. 4, 1. Februar 2012 (2012-02-01), Seiten 1040-1047, XP028478868, ISSN: 0091-6749, DOI: 10.1016/J.JACI.2012.02.005 [gefunden am 2012-02-10]
• DATABASE BIOSIS [Online] BIOSCIENCES INFORMATION SERVICE, PHILADELPHIA, PA, US; November 2013 (2013-11), LAU S ET AL: "What did we learn from farm studies: state of the art clinical studies with bacterial lysates for allergy prevention", XP002780776, Database accession no. PREV201400110752 & ALLERGOLOGIE, Bd. 36, Nr. 11, November 2013 (2013-11), Seiten 481-485, ISSN: 0344-5062, DOI: 10.5414/ALX01633
• DATABASE BIOSIS [Online] BIOSCIENCES INFORMATION SERVICE, PHILADELPHIA, PA, US; Juni 2014 (2014-06), LAU S: "Oral application of bacterial lysate in infancy diminishes the prevalence of atopic dermatitis in children at risk for atopy", XP002780777, Database accession no. PREV201400344212 & BENEFICIAL MICROBES, Bd. 5, Nr. 2, Juni 2014 (2014-06), Seiten 147-149, ISSN: 1876-2883(print), DOI: 10.3920/BM2013.0007

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 492 089 B1

• JOHN PENDERS ET AL: "Establishment of the intestinal microbiota and its role for atopic dermatitis in early childhood", JOURNAL OF ALLERGY AND CLINICAL IMMUNOLOGY, Bd. 132, Nr. 3, 1. September 2013 (2013-09-01), Seiten 601-607.e8, XP055472379, AMSTERDAM, NL ISSN: 0091-6749, DOI: 10.1016/j.jaci.2013.05.043

**Beschreibung**

HINTERGRUND

Die wichtigste Funktion der Haut ist die Barrierefunktion, d.h. der Schutz des Organismus vor dem Austrocknen und vor äußeren Einwirkungen. Die äußere Hautschicht (Epidermis) spielt dabei naturgemäß eine besondere Rolle. Sie ist die Kontaktfläche zur Umwelt und muss stets flexibel und geschmeidig sein. Hierbei spielt die Hautfeuchtigkeit eine ganz wesentliche Rolle. Sie wirkt in der Epidermis wie ein Weichmacher in einem System aus Lipid- und Proteinstrukturen, welche im Prozess der Neubildung der Haut stetig nachgebildet werden.

Es gibt verschiedene Erkrankungen der Haut, die zu einer zu trockenen Haut führen, so dass die Haut ihre Barrierefunktion nicht mehr wahrnehmen kann.

Gewöhnlich basieren pharmazeutische und kosmetische Ansätze die Feuchtigkeit der Haut zu verbessern auf der Verwendung von Stoffen, die dem geschädigten Gewebe durch eine Feuchtigkeit verursachende Zusammensetzung wieder Feuchtigkeit zu zuführen oder/und das Abtrocknen von Feuchtigkeit mittels einer Schutzschicht verhindern.

Daher liegt der Erfindung die Aufgabe zugrunde, eine neue Zusammensetzung bereitzustellen, die bei der Therapie, supportiven Therapie oder Prophylaxe dermatologischer Zustände und Erkrankungen zur Anwendung kommt.

ZUSAMMENFASSUNG

Diese Aufgabe wird durch den Gegenstand der Ansprüche gelöst.

Offenbart ist daher eine pharmazeutische Zusammensetzung, enthaltend eine wirksame Menge einer Mischung aus inaktivierten *Escherichia coli* und *Enterococcus faecalis* sowie pharmazeutisch verträgliche Hilfs- und/oder Trägerstoffe zur Verwendung bei der Therapie, supportiven Therapie oder Prophylaxe dermatologischer Zustände und Erkrankungen, wobei die Zusammensetzung in einer topisch applizierbaren Form vorliegt und zur äußeren Applikation auf die Haut bestimmt ist.

*Escherichia coli* und *Enterococcus faecalis können* als Lysat vorliegen.

Die Mischung aus *Escherichia coli* und *Enterococcus faecalis* kann in einer Menge von 1-95 Gew.%, 20-80 Gew.%, 40-60 Gew.%, oder 45-55 Gew.% in der Gesamtzusammensetzung vorliegen.

*Escherichia coli* und *Enterococcus faecalis* können in einem Verhältnis zwischen 0.5:1.5 und 1.5:0.5, 0.75:1.25 und 1.25:0.75, oder 1.15:0.85 und 0.85:1.15, oder 1.05:0.95 und 0.95:1.05 in der Mischung vorliege

*Escherichia coli* und *Enterococcus faecalis* können jeweils in einer Zellzahl von 0,5 x $10^7$ bis 10x $10^7$, 1,0 x $10^7$ bis 7x $10^7$, oder 1,5 x $10^7$ bis 4,5x $10^7$ pro 100 g der Gesamtmasse vorliegen.

Die dermatologische Erkrankung oder der dermatologische Zustand können ausgewählt werden aus entzündlichen Hauterkrankungenund Neurodermitis.

Die Zusammensetzung kann als Salbe, Flüssigkeit, Emulsion oder Lösung vorliegen.

Weiterhin ist eine Verwendung einer Zusammensetzung enthaltend eine wirksame Menge einer Mischung aus inaktiviertem *Escherichia coli* und *Enterococcus faecalis* zur äußerlichen kosmetischen Anwendung auf der Haut, wobei die Zusammensetzung in einer topisch applizierbaren Form vorliegt, offenbart.

*Enterococcus faecalis* und *Escherichia coli* können in der Zusammensetzung als Lysat vorliegen.

Die Mischung aus *Escherichia coli* und *Enterococcus faecalis* in der Zusammensetzung kann in einer Menge von 1-95 Gew.%, 20-80 Gew.%, 40-60 Gew.%, oder 45-55 Gew.% in der Gesamtzusammensetzung vorliegen.

*Escherichia coli* und *Enterococcus faecalis* können in der Zusammensetzung in einem Verhältnis zwischen 0,5:1,5 und 1,5:0,5, 0,75:1,25 und 1,25:0,75, oder 1,15:0,85 und 0,85:1,15, oder 1,05:0,95 und 0,95:1,05 vorliegen.

*Escherichia coli* und *Enterococcus faecalis* können in der Zusammensetzung jeweils in einer Zellzahl von 0,5 x $10^7$ bis 10x $10^7$, 1,0 x $10^7$ bis 7x $10^7$, oder 1,5 x $10^7$ bis 4,5x $10^7$ pro 100 g der Gesamtmasse vorliegen.

Die kosmetische Anwendung kann ausgewählt sein aus trockener Haut oder transepidermalem Wasserverlust.

Die Zusammensetzung kann als Salbe, Flüssigkeit, Emulsion oder Lösung vorliegen.

KURZE BESCHREIBUNG DER ZEICHNUNGEN

Abb. 1: Bestimmung der IL-8 Ausschüttung in HaCaT-Zellen nach Behandlung mit 10 ng/mL TNF-alpha (Kontrollen ohne TNF-alpha) und Inkubation mit der Testsubstanz (PSF) oder Hydrocortison (HC) für 72 h (wenn nicht anders gekennzeichnet), MW $\pm$ SEM, n = 3 (jeweils in 3-facher Ausfertigung).

Abb. 2: Relative IL-8-Ausschüttung zur Kontrolle mit TNF-alpha (72 h) in HaCaT-Zellen nach Behandlung mit 10 ng/mL TNF-alpha (Kontrollen ohne TNF-alpha) und Inkubation mit der Testsubstanz (PSF) oder Hydrocortison (HC) für 72 h (wenn nicht anders gekennzeichnet), MW $\pm$ SEM, n = 3 (jeweils in 3-facher Ausfertigung).

Abb. 3: Bestimmung der IL-6 Ausschüttung in HaCaT-Zellen nach Behandlung mit 10 ng/mL TNF-alpha (Kontrollen ohne TNF-alpha) und Inkubation mit der Testsubstanz (PSF) oder Hydrocortison (HC) für 72 h (wenn nicht anders

gekennzeichnet), MW $\pm$ SEM, n = 3 (jeweils in 3-facher Ausfertigung).

Abb. 4: Relative IL-6-Ausschüttung zur Kontrolle mit TNF-alpha (72 h) in HaCaT-Zellen nach Behandlung mit 10 ng/mL TNF-alpha (Kontrollen ohne TNF-alpha) und Inkubation mit PSF oder Hydrocortison (HC) für 72 h (wenn nicht anders gekennzeichnet), MW $\pm$ SEM, n = 3 (jeweils in 3-facher Ausfertigung).

DETAILLIERTE BESCHREIBUNG

Unter einer Mischung inaktivierter Bakterien wird eine Mischung von Bakterien verstanden, bei der die Bakterien nicht viabel sind, also keine Stoffwechsel mehr durchführen und/oder nicht zur Vermehrung fähig sind. Inaktivierte Bakterien können durch Bestrahlung mit radioaktiver oder ultravioletter Strahlung, Erhitzung, Gefrieren, Behandlung mit die Bakterienhülle perforierenden Substanzen, wie Detergenzien oder Salzen, oder Lyse, z.B durch Exposition gegenüber einem Druckabfall, der die Zellmembran zerstört (z.B. French Press) erhalten werden.

Ein Lysat kann durch Lyse hergestellt werden, indem eine Flüssigkultur enthaltend Bakterien mit einer gewünschten Zellzahl für 10-20, 12-18, 13-17, oder 14 bis 16 Minuten homogenisiert wird, zum Beispiel durch Rühren bei 50-150, 70-130, oder 80-120 UpM. Die homogenisierte Flüssigkultur wird dann einer üblichen Autoklaven-Behandlung unterzogen, zum Beispiel, für mindestens 20 Minuten (z.B. 20-40 Min, 20-30, oder 20-25 Minuten) bei mindestens 121°C (zum Beispiel, 121°C-130°C, 121°C-125°C, oder 121°C-123°C) erhitzt, d.h. sterilisiert. Danach wird das erhaltene Lysat abgekühlt (z.B. auf 18-20°C).

Unter einer wirksamen Menge wird eine Menge eines Inhaltsstoffes angesehen, die ausreicht einen gewünschten oder therapeutischen Effekt zu erzielen.

Wird einer zahlenmäßigen Angabe der Ausdruck "ungefähr" vorangestellt, so wird hiermit ein Wertebereich von +/- 20%, vorzugsweise +/- 10%, oder besonders bevorzugt +/- 5% um die zahlenmäßigen Angabe herum bezeichnet.

Die Kapitelüberschriften dienen zum Vereinfachen des Verständnisses der Offenbarung, und trennen nicht eine Offenbarung von einer anderen Offenbarung ab. Insbesondere kann jede der einer Kapitelüberschrift folgenden Offenbarung mit einer oder mehreren anderen einer Kapitelüberschrift folgenden Offenbarungen kombiniert werden.

Zusammensetzungen

Offenbart wird eine Zusammensetzung, eine wirksame Menge einer Mischung aus inaktivierten *Escherichia coli* und *Enterococcus faecalis* umfasst. Die Zusammensetzung kann eine pharmazeutische Zusammensetzung sein.

Vorzugsweise ist *Escherichia coli E. coli* DSM 17252.

Vorzugsweise ist *Enterococcus faecalis Enterococcus faecalis* DSM 16440.

Vorzugsweise ist die Mischung eine Mischung aus E. coli DSM 17252 und *Enterococcus faecalis* DSM 16440.

Die Zusammensetzung kann als Lysat vorliegen. Vorzugsweise enthält das Lysat alle löslichen und unlöslichen Bestandteile der Bakterien und liegt daher als wässrige Suspension vor. Das Lysat kann beispielsweise, wie oben beschrieben, gewonnen werden.

Die Mischung aus *Escherichia coli* und *Enterococcus faecalis* kann in einer Menge von 1-95 Gew.%, 10-90 Gew.%, 20-80 Gew.%, 30-70 Gew.%, 40-60 Gew.%, 45-55 Gew.%, 50-54 Gew.%, oder 52-53 Gew.% in der der Gesamtzusammensetzung vorliegen.

*Escherichia coli* und *Enterococcus faecalis* können in einem Verhältnis zwischen 0,5:1.5 und 1,5:0.5, 0,75:1,25 und 1,25:0,75, oder 1,15:0,85 und 0,85:1,15, oder 1,05:0,95 und 0,95:1,05 in der Mischung vorliegen.

*Escherichia coli* und *Enterococcus faecalis* können jeweils in einer Zellzahl von 0,5 x $10^7$ bis 10x $10^7$, 1,0 x $10^7$ bis 7x $10^7$, oder 1,5 x $10^7$ bis 4,5x $10^7$ pro 100 g der Gesamtmasse vorliegen. Der Ausdruck Zellzahl bezieht sich bei einem zellfreien Lysat auf die Menge der Zellen, die eingesetzt wurden, um das Lysat herzustellen.

Die pharmazeutische Zusammensetzung umfasst pharmazeutisch verträgliche Hilfs- und/oder Trägerstoffe.

Die Zusammensetzung kann daher optional enthalten (Bezeichnung gemäß "International Nomenclature of Cosmetic Ingredients", INCI, wobei übliche deutsche Produktbezeichnungen, soweit geläufig, ebenfalls angegeben sind):

Simmondsia Chinensis Seed Oil, insbesondere 0,5 bis 10 Gew.% oder ungefähr 3,00 Gew.% Simmondsia Chinensis Seed Oil (natives Jojobawachs),

Caprylic/Capric Triglyceride, insbesondere 1-20 Gew.% oder ungefähr 16,80 Gew.% Caprylic/Capric Triglyceride (mittelkettige Triglyceride),

Cera Alba, insbesondere 0,1 bis 3,0 Gew.% oder ungefähr 0,50 Gew.% Cera Alba (Bienenwachs),

Hydrogenated Castor Oil, insbesondere 0,1 bis 3,0 Gew.% oder ungefähr 0,80 Gew.% Hydrogenated Castor Oil,

Cetyl PEG/PPG-10/1 Dimethicone, insbesondere 1,0 bis 4,0 Gew.% oder ungefähr 2,0 Gew.% Cetyl PEG/PPG-10/1 Dimethicone (Abil EM 180),

Cetyl Palmitate, inbesondere 0,1 bis 3,0 Gew.% oder ungefähr 0,3 Gew.% Cetyl Palmitate,

eine Mischung aus einer Mischung aus Glyceryl Dibehenate, Tribehenin, Glyceryl Behenate, Squalane, Ceramide 3, Ceramide 3B, Cermamide 6, Cholsterol, insbesondere 0,1 bis 10,0 Gew.% oder ungefähr 0,5 Gew. einer Mischung aus Glyceryl Dibehenate, Tribehenin, Glyceryl Behenate, Squalane, Ceramide 3, Ceramide 3B, Cermamide 6, Cholsterol, Phytosphingosine (wobei diese Mischung ungefähr 56,0-75,0 Gew.% Squalane, ungefähr 5,0-15,0 Gew.% einer Mischung aus Glyceryl Dibehenate, Tribehenin, Glyceryl Behenate im Gewichtsverhältnis von ungefähr 1:1:1 enthält, ungefähr 1,0-5,0 Gew.% einer Mischung aus Ceramide 3 und Ceramide 3B im Gewichtsverhältnis von ungefähr 1:1 enthält, ungefähr 0,1-1,0 Gew.% Cermamide 6, und ungefähr 5,0-15,0 Gew.% einer Mischung aus Cholsterol und Phytosphingosine im Gewichtsverhältnis von ungefähr 1:1 enthält, wobei sie alle Anteil zu 100 Gew.% ergänzen),

Oenothera Biennis Oil, insbesondere 0,5 bis 10 Gew.% oder ungefähr 2,00 Gew.% Oenothera Biennis Oil (enthaltend optional Tocopherol in einer Menge ausreichend Oxidation zu vermeiden, mit Vitamin E stabilisiertes Nachtkerzenöl),

Squalane, insbesondere 1,0 bis 20 Gew.% oder ungefähr 6,0 Gew.% Squalane (Squalan, diese Menge an Squalane ist zusätzlich zu anderen Anteilen an Squalane enthalten, die in der Zusammensetzung enthalten sind),

Prunus Amygdalus Dulcis Oil, insbesondere 0,5 bis 20 Gew.% oder ungefähr 2,50 Gew.% Prunus Amygdalus Dulcis Oil (Mandelöl),

Persea Gratissima Oil, insbesondere 0,5 bis 10 Gew.% oder ungefähr 2,00 Gew.% Persea Gratissima Oil (raffiniertes Avocadoöl),

Tocopherolacetate, insbesondere 0,1 bis 5 Gew.% oder ungefähr 0,5 Gew.% Tocopherolacetate (Vitamin-E-Acetat),

Butyrospermum Parkii Butter, insbesondere 0,1 bis 5 Gew.% oder ungefähr 0,5 Gew.% Butyrospermum Parkii Butter (Sheabutter),

Pentylene Glycol, insbesondere 1,0 bis 5,0 Gew.% oder ungefähr 4,0 Gew.% Pentylene Glycol,

Glycerin, insbesondere 1,0 bis 5,0 Gew.% oder ungefähr 4,0 Gew.% Glycerin (Glycerin 99%),

Sodium Hyaluronate, insbesondere 0,01 bis 0,5 Gew.% oder ungefähr 0,10 Gew.% Sodium Hyaluronate (Na-Hyaluronat),

Panthenol, insbesondere 0,1 bis 5,0 Gew.% oder ungefähr 1,0 Gew.% Panthenol (75% Dexpanthenol),

Betain, 0,1 bis 5,0 Gew.% oder ungefähr 0,3 Gew.% Betain,

Magnesium Sulfate, insbesondere 0,1 bis 3,0 Gew.% oder ungefähr 0,8 Gew.% Magnesium Sulfate (Magnesiumsulfat Heptahydrat),

Sodium Lactate, insbesondere 0,05 bis 1,00 Gew.% oder ungefähr 0,10 Gew.% 50% Sodium Lactate (Na-Lactat 50%),

Sodium Gluconate, insbesondere 0,05 bis 3,00 Gew.% oder ungefähr 0,10 Gew.% Sodium Gluconate, oder/und

Lactic Acid, insbesondere 0,01 bis 1,00 Gew.% Lactic Acid (90%) (Milchsäure 90%).

Vorzugsweise enthält die Zusammensetzung alle oben genannten Hilfsstoffe. Alle Kombinationen aus 1, 2, 3, 4, 5, 6, 7, 8, 9, 10, 11, 12, 13, 14, 15, 16, 17, 18, 19, 20, 21, und 22 der oben genannten Hilfsstoffen sind ebenfalls offenbart.

Vorzugsweise enthält die Zusammensetzung weniger als 10, 9, 8, 7, 6, 5, 4, 3, 2, 1, 0,1, 0,001 oder 0,0001 Gew.% ungesättigte Fettsäuren oder 0 Gew.% ungesättigten Fettsäuren in freier oder/und veresterter Form. Ungesättigte Fettsäuren sind Fettsäuren, die mindestens eine Doppelbindung enthalten, wobei Ceramide in dieser Offenbarung nicht zu den ungesättigten Fettsäuren gezählt werden und daher in der Zusammensetzung enthalten sein können.

Der pH der Zusammensetzung liegt zwischen 4 und 6 oder 4.5 und 5. Der pH wird vorzugsweise durch Beigabe einer pharmazeutisch verträglichen Säure, vorzugsweise einer organischen Säure eingestellt. Besonders bevorzugt ist die Säure zum Einstellen des pH Lactic Acid (Milchsäure).

Dermatologische Zustände und Erkrankungen

Die pharmazeutische Zusammensetzung wird zur Verwendung bei der Therapie, supportiven Therapie oder Prophylaxe dermatologischer Zustände und Erkrankungen eingesetzt. Vorzugsweise sind die dermatologische Erkrankung und der dermatologische Zustand ausgewählt aus trockener Haut, transepidermalem Wasserverlust, entzündlichen Hauterkrankungen, Neurodermitis.

Insbesondere ist die pharmazeutische Zusammensetzung geeignet zur Behandlung von Hauterkrankungen oder Hautzuständen, die sich durch eine (optional erhöhte) Freisetzung von Interleukin-8 auszeichnen. Eine erhöhte Freisetzung von Interleukin-8 ist eine Freisetzung von Interleukin-8, die mindestens 5%, 10%, 20%, 30%, 40%, 50%, oder 100% über der Ausschüttung von Interleukin-8, die bei einem Subjekt mit gesunder Haut auftritt. Die erfindungsgemäße Zusammensetzung kann die Freisetzung von Interleukin-8 um mindestens 10%, 20%, 30%, 40%, 50%, oder 100% reduzieren. Interkeukin-8 wird in entzündetem Gewebe freigesetzt und wird daher bei den oben genannten dermatologische Erkrankungen und dermatologische Zuständen freigesetzt. Eine Abnahme der Freisetzung von Interkeukin-8 ist daher kennzeichnend für eine Abnahme der Entzündung im betroffenen Gewebe.

Der dermatologische Zustand "trockene Haut" kann durch einen zu geringen Fett- und/oder Feuchtigkeitsgehalt gekennzeichnet sein und äußert sich durch ein glanzloses, fahles Aussehen, eine geringe Elastizität, ein Spannungsgefühl und Juckreiz.

Transepidermalem Wasserverlust (TEWL) bezeichnet die Abdunstung von Wasser von der Haut ohne Einbeziehung des Wasserverlustes durch Schwitzen, da über die Haut Wasser auf zwei Wegen abgegeben werden kann, durch Schwitzen und durch passive Diffusion. Der Vorgang der passiven Diffusion durch die Haut wird als transepidermaler Wasserverlust bezeichnet. Der TEWL ist stark von der Intaktheit des Stratum corneums abhängig. Somit ist der TEWL ein Wert mit dem der Hautzustand charakterisiert bewertet werden kann. Die Erfindungsgemäße Zusammensetzung ist zur Verringerung des TEWL geeignet, insbesondere zur Verringerung des TEWL um mindestens 5%, 10%, 15%, 20%, 25%, 30%, 40%, oder 50%.

Zur Bestimmung des TEWL kann jedes dafür bekannte Verfahren eingesetzt werden, z.B. unventilierte Kammern, ventilierte Kammern, der Dampfdruckgradient (z.B. mit einem Tewameter) oder ein Corneometer eingesetzt werden.

Beim Corneometerprinzip des Corneometers wird durch eine Kapazitätsmessung die Hautfeuchtigkeit der "äußeren Schicht", der Oberhaut (Stratum corneum) bestimmt. Diesem Prinzip liegt die Tatsache der unterschiedlichen Dielektrizitätskonstanten von Wasser und anderen Stoffen zugrunde. Ein entsprechend geformter Messkondensator reagiert auf die in sein Messvolumen eingebrachten Proben mit unterschiedlichen Kapazitätsänderungen, die vom Gerät vollautomatisch erfasst und ausgewertet werden. Die mit Spezialglas beschichtete aktive Sonde wird auf die zu messende Hautstelle gedrückt und nach 1 Sekunde erscheint auf der Anzeige der Corneometermesswert, also der Grad der Feuchtigkeit auf der Hautoberfläche. Durch eine besondere Konstruktion wird sichergestellt, dass die aktive Stirnfläche der Sonde jeweils mit konstanter Kraft auch auf alle unzugänglichen Hautstellen gedrückt wird.

Das Corneometer besteht aus einem Pultgehäuse und dem dazugehörigen Messfühler. Dieser ist mit dem Pultgehäuse durch ein Wendelkabel mit Spezialstecker verbunden. Auf dem im Pultgehäuse befindlichen Anzeigefeld wird der Messwert als maximal dreistellige Zahl dargestellt. Das Anzeigefeld erfüllt außerdem noch zusätzliche Informationsfunktionen.

Der Messsensor ist quadratisch ausgebildet. Seine spezialglasbeschichtete aktive Stirnfläche ist axial beweglich und hat einen Hub von wenigstens 3 mm. Das Messprinzip verlangt ein planes Anliegen der Stirnfläche bei konstantem Andruck. Um dies möglichst reproduzierbar zu gewährleisten, wurde die Stirnseite des Messkopfes sehr klein (7 x 7 mm) ausgelegt. Der innere bewegliche Teil - die aktive Stirnfläche - wird durch eine Feder mit jeweils 3,5N auf die Haut gedrückt.

Das Corneometer kann vollautomatisch arbeiten. Zur Durchführung einer Messung wird der Messkopf auf die zu messende Stelle der Haut gedrückt. Nach einer Sekunde wird der Messwert angezeigt.

Der Anzeigewert des Corneometers gibt den Grad der Feuchtigkeit auf der Hautoberfläche an, z.B. vor und nach Behandlungen der Haut mit kosmetischen oder pharmazeutischen Erzeugnissen, d.h. das Gerät zeigt den Zustand bzw. die Veränderung der Feuchtigkeit der Hautoberfläche an.

Die Messungen können in einem konstanten Zeitfenster nach der Applikation der Zusammensetzung durchgeführt.

Entzündliche Hauterkrankungen sind Hauterkrankungen ausgelöst durch Allergene, Urtikaria, Mastozytose, Ekzeme, Dermatitis, Arzneimittelexanthem, Lichen, Erythema nodosum, Juveniles Xanthogranulom, Granuloma anulare, Pyoderma gangraenosum, und/oder Necrobiosis lipoidica. Insbesondere umfassen die Ekzeme atopische Ekzeme, auch bezeichnet als Neurodermitis, atopische Dermatitis, und endogenes Ekzem.

Form der Applikation

Die Zusammensetzung kann als Salbe, Flüssigkeit, Emulsion oder Lösung vorliegt.

Die Zusammensetzung kann topisch appliziert werden, insbesondere durch äußere Applikation der Zusammensetzung auf die Haut, wobei die Haut nicht die Schleimhäute, insbesondere der Nase umfasst.

Die Applikation kann über einen Zeitraum von 1, 2, 3, 4, 5, oder 7 Tagen erfolgen. Die Applikation kann über einen Zeitraum von 1, 2, 3, 4, 5, oder 7 Wochen erfolgen, vorzugsweise über einen Zeitraum von 4 Wochen. Die Applikation kann 1, 2, 3, 4, oder 5 Mal pro Tag erfolgen, vorzugsweise 2 Mal pro Tag. Vorzugsweise erfolgen bei mehreren Applikationen pro Tag die Applikationen in zeitlich ungefähr gleichen Abständen.

Besonders bevorzugt erfolgt die Applikation der Zusammensetzung über einen Zeitraum von 4 Wochen, bei täglicher zweifacher Applikation (vorzugsweise im Abstand von 12 h).

Herstellungsverfahren

Beschrieben ist Verfahren zur Herstellung einer pharmazeutischen Zusammensetzung oder kosmetischen Zusammensetzung, wobei eine wirksame Menge an inaktiviertem *Escherichia coli* und *Enterococcus faecalis* mit pharmazeutisch oder kosmetisch verträgliche Hilfs- und/oder Trägerstoffe vermischt wird, um die genannte dermatologisch wirksame pharmazeutische oder kosmetische Zusammensetzung zu erhalten.

Vorzugsweise ist die erhaltene Zusammensetzung eine Emulsion.

Die Emulsion kann durch bekannte Verfahren hergestellt werden. Zum Beispiel kann die Emulsion über folgendes Verfahren hergestellt werden, wobei die eingesetzten Mengen, den oben angegebenen Mengen für die Mischung enthaltend inaktiviertem *Escherichia coli* und *Enterococcus faecalis* und für die Hilfsstoffe entsprechen:

Sodium Hyaluronate wird in Pentylene Glycol und Glycerin dispergiert, wodurch sich eine Phase C ergibt;

die Mischung enthaltend inaktiviertem *Escherichia coli* und *Enterococcus faecalis* wird als wässrige Lösung mit Panthenol, Betain, Magnesium Sulfate, Sodium Lactate, Sodium Gluconate, und Lactic Acid vermengt ergebend eine Phase D; anschließend wird Phase D zur Phase C gegeben, wodurch sich eine Phase E ergibt;

Phase E wird gerührt bis das Sodium Hyaluronate (klar) gelöst ist;

Phase E wird mit einer organischen Säure, z.B. Lactic Acid auf einen pH von 4,0 bis 5,5 oder 4,5 bis 5,0 eingestellt.

Phase E wird auf ungefähr 75°C erwärmt;

Es wird eine Phase A durch Mischen der Bestandteile Simmondsia Chinensis Seed Oil, Caprylic/Capric Triglyceride, Cera Alba, Hydrogenated Castor Oil, Cetyl PEG/PPG-10/1 Dimethicone, Cetyl Palmitate, Glyceryl Dibehenate, Tribehenin, Glyceryl Behenate, Squalane, Ceramide 3, Ceramide 3B, Cermamide 6, Cholsterol, Phytosphingosine hergestellt; Phase A wird auf ungefähr 75°C erwärmt;

Es wird Phase B durch Mischen der Bestandteile Oenothera Biennis Oil, Squalane, Prunus Amygdalus Dulcis Oil, Persea Gratissima Oil, Tocopherolacetate, Butyrospermum Parkii Butter hergestellt; Phase B wird auf ungefähr 50°C erwärmt;

Erwärmte Phase E wird zu Phase A gegeben und vermischt ergebend eine Phase AE; Erwärmte Phase B wird zu AE gegeben und homogenisiert, wodurch sich eine Wasser-in-Öl Emulsion ergibt;

Die erhaltene Emulsion wird unter Rühren auf ungefähr 30°C (maximal 30°C) abgekühlt und auf dieser Temperatur gehalten;

Die Emulsion wird bei ungefähr 30°C (maximal 30°C) für ungefähr 5 Minuten homogenisiert, wodurch die erfindungsgemäße Zusammensetzung als Emulsion erhalten wird.

Die Mischschritte können in einem Rührwerk bei ungefähr 75 rpm durchgeführt. Die Homogenisierungsschritte werden in einem Homogenisator bei ungefähr 19000 rpm durchgeführt.

Kosmetische Verfahren

Beschrieben wird ein kosmetisches Verfahren zur Erhöhung der Elastizität und/oder Feuchtigkeit der Haut mit der oben beschriebenen erfindungsgemäßen Zusammensetzung. Dieses ist geeignet bei Personen, deren Haut nicht in irgendeiner Weise pathologisch verändert ist.

Ferner beschrieben wird, dass bei dem kosmetischen Verfahren die erfindungsgemäße Zusammensetzung topisch appliziert werden kann, insbesondere durch äußere Applikation der Zusammensetzung auf die Haut, wobei die Haut nicht die Schleimhäute, insbesondere der Nase umfasst.

Die Applikation kann über einen Zeitraum von 1, 2, 3, 4, 5, oder 7 Tagen erfolgen. Die Applikation kann über einen Zeitraum von 1, 2, 3, 4, 5, oder 7 Wochen erfolgen, vorzugsweise über einen Zeitraum von 4 Wochen. Die Applikation kann 1, 2, 3, 4, oder 5 Mal pro Tag erfolgen, vorzugsweise 2 Mal pro Tag. Vorzugsweise erfolgen bei mehreren Applikationen pro Tag die Applikationen in zeitlich ungefähr gleichen Abständen.

Besonders bevorzugt erfolgt die Applikation der Zusammensetzung über einen Zeitraum von 4 Wochen, bei täglicher zweifacher Applikation (vorzugsweise im Abstand von 12 h).

BEISPIELE

Beispiel 1: Wirkung einer Mischung enthaltend inaktivierte *Escherichia coli* und *Enterococcus faecalis* auf die Ausschüttung von Interleukinen in HaCaT Zellen

Zum Nachweis des Einsatzes der erfindungsgemäßen Zusammensetzung zur Pflege bei entzündlichen Hauterkrankungen wurden Untersuchungen zur Reduzierung der Zytokin-Ausschüttung durchgeführt.

Exemplarisch wurde hier die Ausschüttung von Interleukin-6 (IL-6) und Interleukin-8 (IL-8) bestimmt. Durch die Stimulation mit TNF-alpha (10 ng/mL) können Interleukine in verschiedenen Zelltypen gebildet werden. Durch Applikation entzündungshemmender Wirkstoffe oder Immunmodulatoren kann die Interleukin-Produktion beeinflusst werden. Die immunmodulierenden Eigenschaften werden an HaCaT-Zellen (Human adult low Calcium high temperature keratinocytes) getestet. Die aus humanen, nicht malignen Keratinozyten (= Normalhaut) gewonnene Zelllinie HaCaT (spontan immortalisiert) entstammt gesunder Haut eines männlichen Patienten und weist im Wesentlichen die Eigenschaften von basalen epidermalen Keratinozyten auf.

Die anti-entzündliche (antiphlogistische) oder immunmodulierende Eigenschaft einer Substanz kann z.B. in vitro an Zelllinien untersucht werden. Bei diesem Versuchsaufbau wird eine Entzündung (Hochregulierung der Interleukin-Produktion) an der Zelllinie durch externe Stimuli induziert.

Testsubstanz 1 hatte die folgende Zusammensetzung:

Natürliche Darmbakterien *Enterococcus faecalis* und *Escherichia coli* in inaktivierter Form. 1 ml Suspension enthält: Bakterienlysat hergestellt aus 1,5 - 4,5 x $10^7$ Zellen von *Escherichia coli* (DSM 17252) und 1,5- 4,5 x $10^7$ Zellen von *Enterococcus faecalis* (DSM 16440).

Die Testsubstanz 1 wird in zwei Verdünnungen auf die stimulierten Zellen appliziert und die Runterregulierung bzw. Modulation der Interleukin-Produktion (IL-6 und IL-8) untersucht. Die Auswertung erfolgt mittels ELISA (Enzyme Linked Immunosorbent Assay). Der anti-entzündliche Wirkstoff Hydrocortison wird als Vergleichssubstanz mit getestet. HaCaT-Zellen werden mit einer Zelldichte von 20.000 Zellen pro Well in einer 24-well-Gewebekulturplatte eingesät; nach 24 Stunden werden die HaCaT-Zellen mit TNF-$\alpha$ (10 ng/mL) stimuliert (Induktion der Interleukin Produktion, z.B. IL-8) und es erfolgt die Zugabe der zu testenden Substanzen. Nach 72 h Inkubation wurden die zwei Entzündungsmarker (IL-8 und IL-6) quantitativ per ELISA ausgewertet.

Für den Nachweis der anti-entzündlichen Aktivität werden drei unabhängige Versuche in dreifacher Ausfertigung durchgeführt.

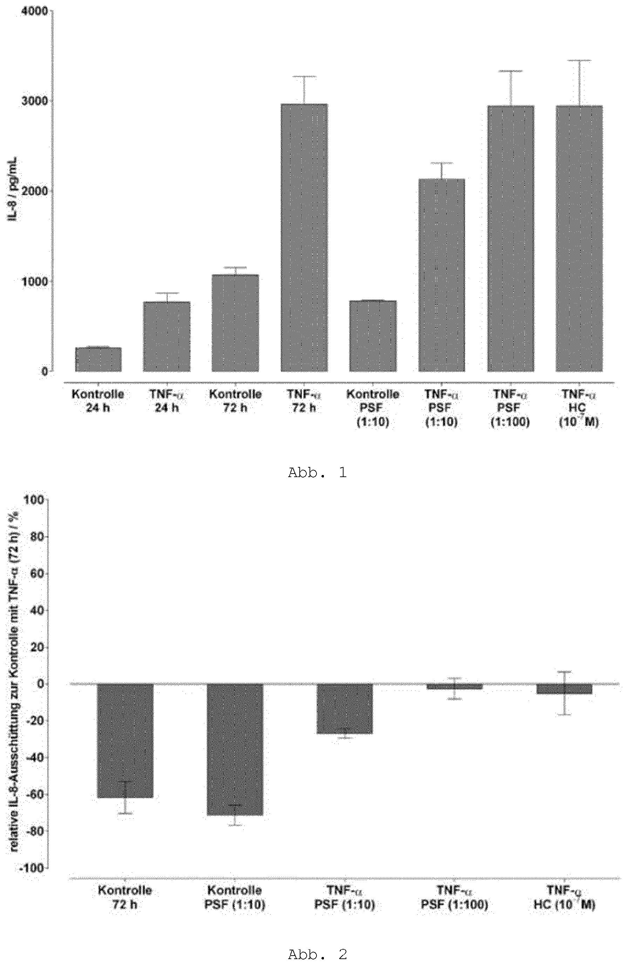

Die Stimulation der HaCaT-Zellen mit TNF-alpha führte zu einer 2,5-fach höheren Ausschüttung von Interleukin-8 im Vergleich zur Kontrolle nach 72 h (Abb. 1 + 2). Die IL-8-Ausschüttung der HaCaT-Zellen inkubiert nur mit der Testsubstanz (1:10, Kontrolle ohne TNF-alpha) war im Mittel um zirka 10% geringer im Vergleich zur Kontrolle bei 72 h. Bei gleichzeitiger Gabe von TNF-alpha und der Testsubstanz (1:10) konnte die IL-8-Ausschüttung um zirka 27% (1:10) reduziert werden im Vergleich zur Kontrolle mit TNF-alpha nach 72 h.

Bei Einsatz der Testsubstanz (1:100) konnte im Mittel keine Modifikation in der IL-8-Ausschüttung im Vergleich zur Kontrolle mit TNF-alpha nach 72 h ermittelt werden.

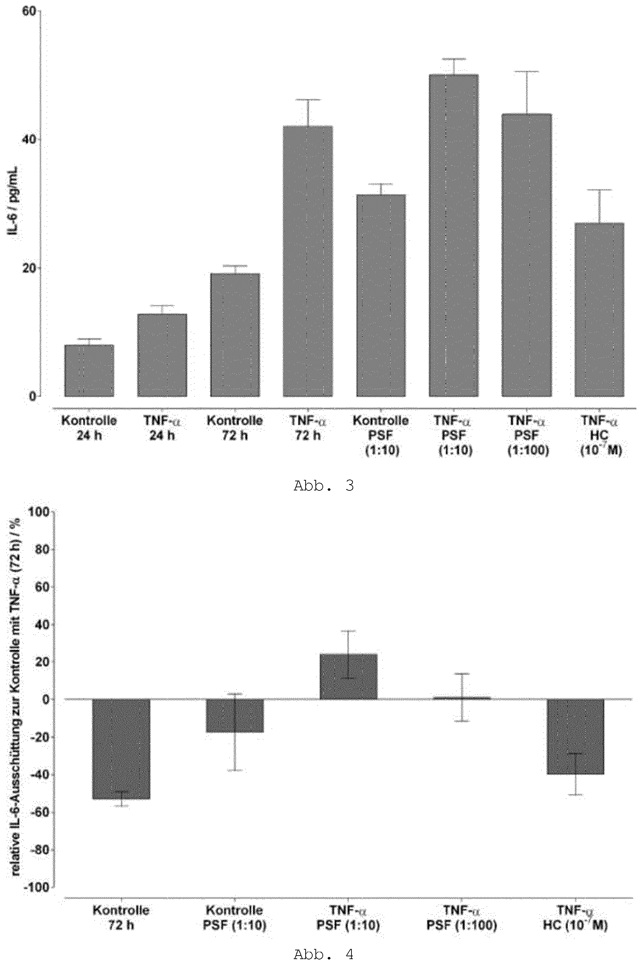

Die Ausschüttung von Interleukin-6 nach Stimulation mit TNF-alpha war deutlich geringer im Vergleich zu Interleukin-8 in HaCaT-Zellen (Abb. 3 + 4). Nach 24 h ohne Zugabe von TNF-alpha (Kontrolle) liegt die IL-6-Ausschüttung unterhalb der Nachweisgrenze. Bei gleichzeitiger Gabe von TNF-alpha und der Substanz (1:10) zeigte sich im Mittel eine leicht gesteigerte IL-6-Ausschüttung um 24% im Vergleich zur Kontrolle mit TNF-alpha nach 72 h.

Die IL-6-Ausschüttung bei der 1:100 Verdünnung der Testsubstanz war vergleichbar mit der behandelten Kontrolle nach 72 h. Anders als bei der IL-8-Ausschüttung führte Hydrocortison ($10^{-7}$ M) zu einer deutlichen Reduktion (40%) der IL-6-Ausschüttung im Vergleich zur behandelten Kontrolle nach 72 h.

Die Untersuchungen zur Beeinflussung des Entzündungsgeschehens durch die Testsubstanz zeigten sowohl bei IL-8 als auch bei IL-6 eine konzentrationsabhängige Modulation der Freisetzung. Bei einer 1:10 Verdünnung der Testsubstanz war die IL-8-Ausschüttung um 27% reduziert, aber die IL-6-Ausschüttung um 24% erhöht im Vergleich zur Kontrolle mit TNF-alpha nach 72 h. Bei einer 1:100 Verdünnung der Testsubstanz lag sowohl die IL-8-Ausschüttung als auch die IL-6-Auschüttung im Bereich der Kontrolle mit TNF-alpha nach 72 h.

Eine Entzündung kann durch Gabe von TNF-alpha ausgelöst werden. TNF-alpha ist ein multifunktionales Zytokin, welches neben Entzündung auch Immunantwort und Apoptose vermittelt. TNF-alpha aktiviert die Immunantwort durch Einleiten der Produktion weiterer Zytokine, die als Entzündungsmarker gelten. IL-6 wird im Körper (in vivo) nach Stimulation durch TNF-alpha gebildet und beeinflusst verschiedene Entzündungsreaktionen. Durch IL-6 wird z.B. die Bildung des C-reaktiven Proteins angeregt, welches häufig als akuter Entzündungsparameter genutzt wird. IL-8 ist ein chemotaktisches Zytokin, welches durch verschiedene Gewebe- und Blutzellen exprimiert wird. Als Entzündungsmediator mobilisiert und aktiviert IL-8 neutrophile Granulozyten und unterstützt ihre Degranulation. Das Zusammenspiel zwischen T-Lymphozyten, neutrophilen Granulozyten und epidermalen Zellen spielt vermutlich eine entscheidende Rolle in der Pathophysiologie von entzündlichen Hauterkrankungen wie z.B. Psoriasis.

Die durchgeführten Untersuchungen an HaCaT-Zellen nach Stimulierung mit TNF-alpha zeigten, dass Testsubstanz 1 konzentrationsabhängig die Interleukin-Ausschüttung in vitro beeinflusst.

Beispiel 2: Wirkung einer Mischung enthaltend inaktiviertem *Escherichia coli* und *Enterococcus faecalis* auf den Transepidermalen Wasserverlust (TEWL) der menschlichen Haut

Der transepidermale Wasserverlust der Haut ist einer der wichtigsten Parameter zur Beurteilung der Schutzfunktion der Haut. Dabei handelt es sich um die Abdunstung von Wasser aus dem Körperinneren ohne Einbeziehung des Wasserverlustes beim Schwitzen. Ein Präparat, das die Hautbarrierefunktion aufrechterhält bzw. verbessert, bewirkt eine Reduzierung des transepidermalen Wasserverlustes. Eine geringe Wasserverdunstung auf der Haut entspricht einer guten Feuchthaltewirkung der Haut.

Die quantitative Bestimmung des transepidermalen Wasserverlustes (TEWL) der Haut wurde mit dem Evaporimeter durchgeführt. Die Messsonde des Evaporimeters besteht aus einem nach oben offenen Rohr mit zwei Temperatur- und Feuchtigkeitssensoren, die mit einem geringen Abstand übereinander befestigt sind. Die beiden Feuchtigkeitssensoren ermitteln den Dampfdruckgradienten unmittelbar in der Diffusionszone über der Haut. Mit EDV-Unterstützung wird die Wasserabgabe in g/hm$^2$ nach dem Fickschen Diffusionsgesetz gemessen:

$$\frac{dm}{dt} = -D \cdot A \cdot \frac{dc}{dx}$$

wobei

dm/dt = Diffusionsstrom

D = Diffusionskoeffizient

A = Fläche

dc/dx = Dichtegradient.

Die Untersuchungen zum Transepidermalen Wasserverlust der Haut wurden mit dem Tewameter® (Courage + Khazaka electronic GmbH) durchgeführt.

Die Messsonde wird ohne Druckausübung auf das zu untersuchende Hautareal aufgelegt, so dass die Ränder des Teflonrohrs mit der Haut abschließen. Insgesamt werden 20 Messzyklen einer Messreihe pro Testfeld durchgeführt, die durch das Gerät registriert und gemittelt werden. Als Testareale diente das Gesicht mit Messfeldern in einer Größenordnung von ca. 3 cm Durchmesser. In diesen Arealen wurde an 3 verschiedenen Stellen gemessen und die Messwerte wurden gemittelt. Die erhaltenen Messwerte werden tabellarisch dargestellt.

20 Probanden/innen zwischen 23 und 66 Jahren stellten das Probandenkollektiv dar. Die Probanden wurden instruiert die Textsubstanz 2 über vier Wochen regelmäßig morgens und abends anzuwenden.

Die Personen weisen im Testareal eine sehr trockene bzw. bis zur Neurodermitis neigende Haut (medizinisch nicht behandlungsbedürftig) auf.

Testsubstanz 2 hatte folgende Zusammensetzung:

3,00 Gew.% Simmondsia Chinensis Seed Oil,

16,80 Gew.% Caprylic/Capric Triglyceride,

0,50 Gew.% Cera Alba,

0,80 Gew.% Hydrogenated Castor Oil,

Cetyl PEG/PPG-10/1 Dimethicone,

0,3 Gew.% Cetyl Palmitate,

0,5 Gew. einer Mischung aus Glyceryl Dibehenate, Tribehenin, Glyceryl Behenate, Squalane, Ceramide 3, Ceramide 3B, Cermamide 6, Cholsterol, Phytosphingosine (wobei die jeweiligen Inhaltsstoffe in jeweils gleichen Anteilen vorliegen),

2,00 Gew.% Oenothera Biennis Oil (stabilisiert mit Tocopherol),

6,0 Gew.% Squalane,

2,50 Gew.% Prunus Amygdalus Dulcis Oil,

2,00 Gew.% Persea Gratissima Oil,

0,5 Gew.% Tocopherolacetate,

0,5 Gew.% Butyrospermum Parkii Butter,

4,0 Gew.% Pentylene Glycol,

4,0 Gew.% Glycerin (99%),

0,10 Gew.% Sodium Hyaluronate,

52,19 Gew.% Testsubstanz 1 (siehe Beispiel 1)

1,0 Gew.% Panthenol,

0,3 Gew.% Betain,

0,8 Gew.% Magnesium Sulfate,

0,10 Gew.% Sodium Lactate,

0,10 Gew.% Sodium Gluconate, oder

Lactic Acid zur Einstellung des pH auf einen Bereich von 4.5 bis 5.

Die folgenden Tabellen geben Durchschnittswerte (in $g/hm^2$) für die TEWL-Messungen im Testareal jeweils vorher und nach 4 Wochen regelmäßiger Anwendung wieder.
Negative Werte ergaben sich, wenn der transepidermale Wasserverlust der Haut von der ersten Messung zur zweiten Messung abgenommen hatte.

Tabelle 1: Darstellung der Messergebnisse (TEWL-Werte) der 20 Testpersonen vor und nach 4 Wochen Anwendung des Präparates und Berechnung der Differenzen vorher / nachher. Rechte Körperhälfte.

|  | Vorher | Nach 4 Wochen | Differenz | Prozentuale Veränderung |
|---|---|---|---|---|
| Durchschnitt | 17,6 | 12,9 | -4,7 | -26,70 |
| Standardabweichung | 27,7 | 22,1 | 4,9 | 30,25 |
| Varianz | 4,8 | 4,5 | 4,0 | 24,21 |

Die durchschnittliche TEWL-Wert-Veränderung (%) durch die Anwendung des Präparates wird über die Einzelergebnisse der 20 Testpersonen gemittelt. Die Differenz der durchschnittlich ermittelten TEWL-Werte beträgt -4,7. Dieser Wert gibt die durchschnittliche Abnahme der Wasserabgabe nach Anwendung des Präparates wieder. Bei einem TEWL-Ausgangswert von durchschnittlich 17,6 entspricht dies einer Abnahme des transepidermalen Wasserverlustes der Haut von ca. -26,70 %.

Tabelle 2: Darstellung der Messergebnisse (TEWL-Werte) der 20 Testpersonen vor und nach 4 Wochen Anwendung des Präparates und Berechnung der Differenzen vorher / nachher. Linke Körperhälfte.

|  | Vorher | Nach 4 Wochen | Differenz | Prozentuale Veränderung |
|---|---|---|---|---|
| Durchschnitt | 16,9 | 13,9 | -3,0 | -17,75 |
| Standardabweichung | 23,0 | 23,7 | 6,2 | 106,90 |
| Varianz | 3,8 | 4,4 | 3,5 | 33,25 |

Die durchschnittliche TEWL-Wert-Veränderung (%) durch die Anwendung des Präparates wird über die Einzelergebnisse der 20 Testpersonen gemittelt. Die Differenz der durchschnittlich ermittelten TEWL-Werte beträgt -3,0. Dieser Wert gibt die durchschnittliche Abnahme der Wasserabgabe nach Anwendung des Präparates wieder. Bei einem TEWL-Ausgangswert von durchschnittlich 16,9 entspricht dies einer Abnahme des transepidermalen Wasserverlustes der Haut von ca. -17,75 %.

Tabelle 3: Darstellung der Messergebnisse (TEWL-Werte) der 20 Testpersonen vor und nach 4 Wochen Anwendung im Kontrollareal (d.h. ohne Applikation der Testsubstanz 2)und Berechnung der Differenzen vorher / nachher.

|  | Vorher | Nach 4 Wochen | Differenz | Prozentuale Veränderung |
|---|---|---|---|---|
| Durchschnitt | 10,3 | 9,8 | -0,5 | -4,85 |
| Standardabweichung | 5,2 | 3,7 | 2,1 | 13,82 |
| Varianz | 27,0 | 13,4 | 4,2 | 190,92 |

Die durchschnittliche TEWL-Wert-Veränderung (%) im unbehandelten Kontrollbereich wird über die Einzelergebnisse der 20 Testpersonen gemittelt. Die Differenz der durchschnittlich ermittelten TEWL-Werte beträgt -0,5. Dieser Wert gibt die durchschnittliche Abnahme der Wasserabgabe ohne Anwendung des Präparates wieder. Bei einem TEWL-Ausgangswert von durchschnittlich 10,3 entspricht dies einer Abnahme des transepidermalen Wasserverlustes der Haut von ca. -4,85 %.

Tabelle 4: Zusammenfassung der durchschnittlichen Veränderungen im Beobachtungszeitraum:

| TEWL Wertveränderung im Testfeld in % (rechte Körperhälfte) | TEWL Wertveränderung im Testfeld in % (linke Körperhälfte) | TEWL Wertveränderung im Kontrollfeld in % |
|---|---|---|
| -26,70 | -17,75 | -4,85 |

Damit lässt sich nachweisen, dass Testsubstanz 2 eine erhebliche Verminderung des transepidermalen Wasserverlustes bei Personen mit ausgeprägt trockener Haut bewirkt.

Beispiel 3: Wirkung einer Mischung enthaltend inaktiviertem *Escherichia coli* und *Enterococcus faecalis* auf die Hautfeuchte

Beim Corneometerprinzip wird durch eine Kapazitätsmessung die Hautfeuchtigkeit der "äußeren Schicht", der Oberhaut (Stratum corneum) bestimmt. Diesem Prinzip liegt die Tatsache der unterschiedlichen Dielektrizitätskonstanten von Wasser und anderen Stoffen zugrunde. Ein entsprechend geformter Messkondensator reagiert auf die in sein Messvolumen eingebrachten Proben mit unterschiedlichen Kapazitätsänderungen, die vom Gerät vollautomatisch erfasst und ausgewertet werden. Die mit Spezialglas beschichtete aktive Sonde wird auf die zu messende Hautstelle gedrückt und nach 1 Sekunde erscheint auf der Anzeige der Corneometermesswert, also der Grad der Feuchtigkeit auf der Hautoberfläche. Durch eine besondere Konstruktion wird sichergestellt, dass die aktive Stirnfläche der Sonde jeweils mit konstanter Kraft auch auf alle unzugänglichen Hautstellen gedrückt wird.

Das Corneometer besteht aus einem Pultgehäuse und dem dazugehörigen Messfühler. Dieser ist mit dem Pultgehäuse durch ein Wendelkabel mit Spezialstecker verbunden. Auf dem im Pultgehäuse befindlichen Anzeigefeld wird der Messwert als maximal dreistellige Zahl dargestellt. Das Anzeigefeld erfüllt außerdem noch zusätzliche Informationsfunktionen.

Der Messsensor ist quadratisch ausgebildet. Seine spezialglasbeschichtete aktive Stirnfläche ist axial beweglich und hat einen Hub von wenigstens 3 mm. Das Messprinzip verlangt ein planes Anliegen der Stirnfläche bei konstantem Andruck. Um dies möglichst reproduzierbar zu gewährleisten, wurde die Stirnseite des Messkopfes sehr klein (7 x 7 mm) ausgelegt. Der innere bewegliche Teil - die aktive Stirnfläche - wird durch eine Feder mit jeweils 3,5N auf die Haut gedrückt.

Das Corneometer arbeitet vollautomatisch. Zur Durchführung einer Messung wird der Messkopf auf die zu messende Stelle der Haut gedrückt. Nach einer Sekunde wird der Messwert angezeigt.

Der Anzeigewert des Corneometers gibt den Grad der Feuchtigkeit auf der Hautoberfläche an, z.B. vor und nach Behandlungen der Haut mit kosmetischen oder pharmazeutischen Erzeugnissen, d.h. das Gerät zeigt den Zustand bzw. die Veränderung der Feuchtigkeit der Hautoberfläche an.

Die Messungen werden in einem konstanten Zeitfenster nach der Produktanwendung durchgeführt.

Die Probanden wurden angewiesen, nur das Testprodukt in dem definierten Testintervall für die Dauer der Testung zu verwenden.

Die Messungen wurden wie folgt durchgeführt:

1. Die Probanden werden 45 Minuten bei einer Temperatur von 22 Grad Celsius und 60% relativer Feuchtigkeit einklimatisiert.

2. Hautmesswerte werden im jeweiligen Testfeld an drei verschiedenen Stellen gemessen. Die gefundenen Werte werden gemittelt.

3. Als Kontrollmessareal dient bei dieser Untersuchung ein nicht behandeltes Hautareal.

4. Messungen fanden vor Anwendungsbeginn und nach 4 Wochen Anwendung der Testzusammensetzung 2 aus Beispiel 2 statt. Die Messungen finden jeweils 10-12 Stunden nach dem letzten Auftrag des bisher genutzten Produktes oder der Testprodukte statt.

Die Hautfeuchtigkeitswerte pro Testfeld und Zeitpunkt werden jeweils gemittelt. Die jeweiligen Werte finden sich in den anhängenden Tabellen.

Über die Probanden, die durch eine laufende Nummer, Alter und Geschlecht spezifiziert sind, wird gemittelt und die Standardabweichung bestimmt.

(Literatur: L. Sachs, "Statistische Methoden", 6.Auflage, Springer Verlag Berlin Heidelberg 1988). delta = Differenzen der Hautfeuchtigkeitswerte delta (%) = mittlere prozentuale Feuchtigkeitsänderung durch die Anwendung, bezogen auf den Ausgangswert

Die entsprechenden Tabellen enthalten die jeweils ermittelten Werte.

Die Personen weisen im Testareal eine sehr trockene bzw. bis zur Neurodermitis neigende Haut (medizinisch nicht behandlungsbedürftig) auf.

Tabelle 5: Hautfeuchtigkeitsmessung im behandelten Areal, rechte Körperhälfte.

|  | Vorher | Nach 4 Wochen | Differenz | Prozentuale Veränderung |
|---|---|---|---|---|
| Durchschnitt | 18,3 | 27,7 | 9,4 | 51,37 |
| Standardabweichung | 4,0 | 7,8 | 6,4 | 40,12 |
| Varianz | 15,7 | 60,8 | 41,4 | 1609,75 |

Tabelle 6: Hautfeuchtigkeitsmessung im behandelten Areal, linke Körperhälfte.

|  | Vorher | Nach 4 Wochen | Differenz | Prozentuale Veränderung |
|---|---|---|---|---|
| Durchschnitt | 17,7 | 27,2 | 9,5 | 53,67 |
| Standardabweichung | 4,1 | 6,3 | 5, 6 | 35,40 |
| Varianz | 16,7 | 40,1 | 31,7 | 1252,89 |

Tabelle 7: Hautfeuchtigkeitsmessung im unbehandelten Kontrollbereich.

|  | Vorher | Nach 4 Wochen | Differenz | Prozentuale Veränderung |
|---|---|---|---|---|
| Durchschnitt | 30,6 | 32,2 | 1,6 | 5,23 |
| Standardabweichung | 5,0 | 4,6 | 2,4 | 8,85 |
| Varianz | 25,4 | 21,5 | 5,9 | 78,29 |

Tabelle 8: Zusammenfassende Auswertung der Hautfeuchtigkeitsmessungen

| Feuchtigkeitsveränd erung im Testfeld in % (rechte Körperhälfte) | Feuchtigkeitsveränd erung im Testfeld in % (linke Körperhälfte) | Feuchtigkeitsveränd erung im Kontrollfeld in % |
|---|---|---|
| 51,37 | 53,67 | 5,23 |

Insgesamt 20 Testpersonen vertrugen im vierwöchigen Anwendungstest nach dermatologisch-klinischen Kriterien die Testsubstanz 2 einwandfrei. Es kam in keinem Fall zu unerwünschten oder gar pathologischen Hautveränderungen im Bereich der Testareale.

Die Corneometriemessung zur Überprüfung der Wirkung der Testsubstanz 2 auf die Hautfeuchtigkeit wurde mit der Corneometersonde CM 825 (Firma Courage+Khazaka) an 20 Probanden durchgeführt.

Die Veränderung der Hautfeuchtigkeitswerte wurde im Testareal vor und nach 4 Wochen regelmäßiger Anwendung der Präparate sowie in einem unbehandelten Kontrollareal bestimmt. Im behandelten Areal zeigte sich eine Verbesserung der Hautfeuchtigkeit von 51,37 % (rechte Körperhälfte) und von 53,67 % (linke Körperhälfte). Die durchschnittliche Hautfeuchtigkeitsveränderung im unbehandelten Hautareal betrug 5,23 %.

Daher zeigt sich eine deutliche Verbesserung der Hautfeuchtigkeit durch Testsubstanz 2.

**Patentansprüche**

1. Pharmazeutische Zusammensetzung, enthaltend eine wirksame Menge einer Mischung aus inaktivierten *Escherichia coli* und *Enterococcus faecalis* sowie pharmazeutisch verträgliche Hilfs- und/oder Trägerstoffe zur Verwendung bei der Therapie, supportiven Therapie oder Prophylaxe dermatologischer Erkrankungen, wobei die Zusammensetzung in einer topisch applizierbaren Form vorliegt und zur äußeren Applikation auf die Haut bestimmt ist.

2. Pharmazeutische Zusammensetzung zur Verwendung nach Anspruch 1, wobei *Escherichia coli* und *Enterococcus faecalis* als Lysat vorliegen.

3. Pharmazeutische Zusammensetzung zur Verwendung nach einem oder mehreren der vorhergehenden Ansprüche, wobei die Mischung aus *Escherichia coli* und *Enterococcus faecalis* in einer Menge von 1-95 Gew.%, 20-80 Gew.%,

40-60 Gew.%, oder 45-55 Gew.% in der der Gesamtzusammensetzung vorliegt.

4. Pharmazeutische Zusammensetzung zur Verwendung nach einem oder mehreren der vorhergehenden Ansprüche, wobei *Escherichia coli* und *Enterococcus faecalis* in einem Verhältnis zwischen 0.5:1.5 und 1.5:0.5, 0.75:1.25 und 1.25:0.75, oder 1.15:0.85 und 0.85:1.15, oder 1.05:0.95 und 0.95:1.05 in der Mischung vorliegen, optional wobei *Escherichia coli* und *Enterococcus faecalis* jeweils in einer Zellzahl von $0,5 \times 10^7$ bis $10 \times 10^7$, $1,0 \times 10^7$ bis $7 \times 10^7$, oder $1,5 \times 10^7$ bis $4,5 \times 10^7$ pro 100 g der Gesamtmasse vorliegen.

5. Pharmazeutische Zusammensetzung zur Verwendung nach einem oder mehreren der vorhergehenden Ansprüche, wobei die dermatologische Erkrankung ausgewählt wird aus entzündlichen Hauterkrankungen und Neurodermitis.

6. Pharmazeutische Zusammensetzung zur Verwendung nach einem oder mehreren der vorhergehenden Ansprüche, wobei die Zusammensetzung als Salbe, Flüssigkeit, Emulsion oder Lösung vorliegt.

7. Verwendung einer Zusammensetzung, enthaltend eine wirksame Menge einer Mischung aus inaktiviertem *Escherichia coli* und *Enterococcus faecalis* zur äußerlichen kosmetischen Anwendung auf der Haut, wobei die Zusammensetzung in einer topisch applizierbaren Form vorliegt.

8. Verwendung nach Anspruch 7, wobei *Escherichia coli* und *Enterococcus faecalis* als Lysat vorliegen.

9. Verwendung nach Anspruch 7 oder 8, wobei die Mischung aus *Escherichia coli* und *Enterococcus faecalis* in einer Menge von 1-95 Gew.%, 20-80 Gew.%, 40-60 Gew.%, oder 45-55 Gew.% in der Gesamtzusammensetzung vorliegt.

10. Verwendung nach einem oder mehreren der Ansprüche 7-9, wobei *Escherichia coli* und *Enterococcus faecalis* in einem Verhältnis zwischen 0,5:1,5 und 1,5:0,5, 0,75:1,25 und 1,25:0,75, oder 1,15:0,85 und 0,85:1,15, oder 1,05:0,95 und 0,95:1,05 vorliegen, optional wobei *Escherichia coli* und *Enterococcus faecalis* jeweils in einer Zellzahl von $0,5 \times 10^7$ bis $10 \times 10^7$, $1,0 \times 10^7$ bis $7 \times 10^7$, oder $1,5 \times 10^7$ bis $4,5 \times 10^7$ pro 100 g der Gesamtmasse vorliegen.

11. Verwendung nach einem oder mehreren der Ansprüche 7-10, wobei die kosmetische Anwendung ausgewählt wird aus trockener Haut oder transepidermalem Wasserverlust.

12. Verwendung nach einem oder mehreren der Ansprüche 7-11, wobei die Zusammensetzung als Salbe, Flüssigkeit, Emulsion oder Lösung vorliegt.

## Claims

1. Pharmaceutical composition, comprenant an effective amount of a mixture of inactivated *Escherichia coli* and *Enterococcus faecalis* and pharmaceutically acceptable excipients and/or vehicles, for use in treatment, supportive treatment or prophylaxis of dermatological diseases, the composition being in a topically applicable form and being intended for external application to the skin.

2. Pharmaceutical composition for use according to claim 1, wherein *Escherichia coli* and *Enterococcus faecalis* take the form of a lysate.

3. Pharmaceutical composition for use according to one or more of the preceding claims, wherein the mixture of *Escherichia coli* and *Enterococcus faecalis* is present in an amount of 1 - 95 % by weight, 20 - 80 % by weight, 40 - 60 % by weight or 45 - 55 % by weight of the total composition.

4. Pharmaceutical composition for use according to one or more of the preceding claims, wherein *Escherichia coli* and *Enterococcus faecalis* are present in the mixture in a ratio of between 0.5:1.5 and 1.5:0.5, 0.75:1.25 and 1.25:0.75, 1.15:0.85 and 0.85:1.15, or 1.05:0.95 and 0.95:1.05, *Escherichia coli* and *Enterococcus faecalis* optionally each being present at a cell count of $0.5 \times 10^7$ to $10 \times 10^7$, $1.0 \times 10^7$ to $7 \times 10^7$, or $1.5 \times 10^7$ to $4.5 \times 10^7$ per 100 g of total mass.

5. Pharmaceutical composition for use according to one or more of the preceding claims, wherein the dermatological disease is selected from inflammatory skin conditions and neurodermitis.

**6.** Pharmaceutical condition for use according to one or more of the preceding claims, wherein the composition takes the form of a salve, liquid, emulsion or solution.

**7.** Use of a composition, containing an effective amount of a mixture of inactivated *Escherichia coli* and *Enterococcus faecalis,* for external cosmetic application to the skin, the composition being in a topically applicable form.

**8.** Use according to claim 7, wherein *Escherichia coli* and *Enterococcus faecalis* take the form of a lysate.

**9.** Use according to claim 7 or claim 8, wherein the mixture of *Escherichia coli* and *Enterococcus faecalis* is present in an amount of 1 - 95 % by weight, 20 - 80 % by weight, 40 - 60 % by weight or 45 - 55 % by weight of the total composition.

**10.** Use according to one or more of claims 7 - 9, wherein *Escherichia coli* and *Enterococcus faecalis* are present in the mixture in a ratio of between 0.5:1.5 and 1.5:0.5, 0.75:1.25 and 1.25:0.75, 1.15:0.85 and 0.85:1.15, or 1.05:0.95 and 0.95:1.05, *Escherichia coli* and *Enterococcus faecalis* optionally each being present at a cell count of $0.5 \times 10^7$ to $10 \times 10^7$, $1.0 \times 10^7$ to $7 \times 10^7$, or $1.5 \times 10^7$ to $4.5 \times 10^7$ per 100 g of total mass.

**11.** Use according to one or more of claims 7 - 10, wherein the cosmetic application is selected from dry skin and transepidermal water loss.

**12.** Use according to one or more of claims 7 - 11, wherein the composition takes the form of a salve, liquid, emulsion or solution.

**Revendications**

**1.** Composition pharmaceutique, contenant une quantité efficace d'un mélange d'*Escherichia coli* et d'*Enterococcus faecalis* inactivés ainsi que des adjuvants et/ou des excipients pharmaceutiquement acceptables pour l'utilisation en thérapie, en thérapie de soutien ou en prophylaxie de maladies dermatologiques, dans laquelle la composition est présente sous une forme topiquement applicable et est destinée à l'application externe sur la peau.

**2.** Composition pharmaceutique pour l'utilisation selon la revendication 1, dans laquelle *Escherichia coli* et *Enterococcus faecalis* sont présents sous forme de lysat.

**3.** Composition pharmaceutique pour l'utilisation selon une ou plusieurs des revendications précédentes, dans laquelle le mélange d'*Escherichia coli* et d'*Enterococcus faecalis* est présent dans une quantité de 1 à 95 % en poids, de 20 à 80 % en poids, de 40 à 60 % en poids, ou de 45 à 55 % en poids par rapport à la composition totale.

**4.** Composition pharmaceutique pour l'utilisation selon une ou plusieurs des revendications précédentes, dans laquelle *Escherichia coli* et *Enterococcus faecalis* sont présents dans le mélange dans une proportion entre 0,5:1,5 et 1,5:0,5, 0,75:1,25 et 1,25:0,75, ou 1,15:0,85 et 0,85:1,15, ou 1,05:0,95 et 0,95:1,05, dans laquelle *Escherichia coli* et *Enterococcus faecalis* sont éventuellement présents avec un nombre de cellules allant de $0,5 \times 10^7$ à $10 \times 10^7$, de $1,0 \times 10^7$ à $7 \times 10^7$, ou de $1,5 \times 10^7$ à $4,5 \times 10^7$ par 100 g de masse totale.

**5.** Composition pharmaceutique pour l'utilisation selon une ou plusieurs des revendications précédentes, dans laquelle la maladie dermatologique est sélectionnée parmi les maladies de peau inflammatoires et la dermatite atopique.

**6.** Composition pharmaceutique pour l'utilisation selon une ou plusieurs des revendications précédentes, dans laquelle la composition est présente sous forme de pommade, de liquide, d'émulsion ou de solution.

**7.** Utilisation d'une composition contenant une quantité efficace d'un mélange d'*Escherichia coli* et d'*Enterococcus faecalis* inactivés pour application cosmétique externe sur la peau, dans laquelle la composition est présente sous une forme topiquement applicable.

**8.** Utilisation selon la revendication 7, dans laquelle *Escherichia coli* et *Enterococcus faecalis* sont présents sous forme de lysat.

**9.** Utilisation selon la revendication 7 ou 8, dans laquelle le mélange d'*Escherichia coli* et d'*Enterococcus faecalis* est présent dans une quantité allant de 1 à 95 % en poids, de 20 à 80 % en poids, de 40 à 60 % en poids, ou de 45 à 55 % en poids par rapport à la composition totale.

10. Utilisation selon une ou plusieurs des revendications 7 à 9, dans laquelle *Escherichia coli* et *Enterococcus faecalis* sont présents dans le mélange dans une proportion entre 0,5:1,5 et 1,5:0,5, 0,75:1,25 et 1,25:0,75, ou 1,15:0,85 et 0,85:1,15, ou 1,05:0,95 et 0,95:1,05, dans laquelle *Escherichia coli* et *Enterococcus faecalis* sont éventuellement présents avec un nombre de cellules allant de $0,5 \times 10^7$ à $10 \times 10^7$, de $1,0 \times 10^7$ à $7 \times 10^7$, ou de $1,5 \times 10^7$ à $4,5 \times 10^7$ par 100 g de masse totale.

11. Utilisation selon une ou plusieurs des revendications 7 à 10, dans laquelle l'application cosmétique est sélectionnée parmi la peau sèche ou la déshydratation transépidermique.

12. Utilisation selon une ou plusieurs des revendications 7 à 11, dans laquelle la composition est présente sous forme de pommade, de liquide, d'émulsion ou de solution.

Abb. 1

Abb. 2

Abb. 3

Abb. 4